# EUROPEAN PATENT APPLICATION

(11) **EP 0 985 449 A1**
(43) Date of publication of application: **15.03.2000**
(21) Application number: 98203053.8
(22) Date of filing: 11.09.1998
(51) Int. Cl.: B01J 31/40, C22B 11/00, C22B 3/26, C07C 51/48, C07C 51/10

(54) **Process to separate rhodium from an organic mixture**

(71) Applicant: DSM N.V., 6411 TE Heerlen (NL)
(72) Inventor: Sielcken, Otto Erik, 6136 BG Sittard (NL); Smeets, Theodorus Maria, 6181 BP Stein (NL)

(57) **Abstract**

Process to separate rhodium from an organic mixture comprising mono-carboxylic acids, dicarboxylic acids, rhodium and a halide compound, wherein the following steps are performed
(1) contacting the organic mixture with an amount of water resulting in two separate phases, a first phase being an aqueous phase comprising rhodium and the dicarboxylic acids and a second phase being an organic phase comprising the mono-carboxylic acids, wherein during contacting the molar ratio of halide and rhodium is higher than 1:1,
(2) separating the aqueous phase from the organic phase,
(3) contacting the aqueous phase comprising the rhodium and the dicarboxylic acids with a sufficient amount of an apolar organic extraction solvent resulting in two separate liquid phases, being an aqueous phase comprising the dicarboxylic acids and an apolar organic phase comprising rhodium.

## Description

The invention relates to a process to separate rhodium from an organic mixture comprising mono-carboxylic acids, dicarboxylic acids, rhodium and a halide compound.

Such a mixture is obtained in a process as described in WO-A-9614287. This publication describes a process in which a reagent, especially an alcohol, is carbonylated to a carboxylic acid in the presence of a catalyst system comprising rhodium and a halide promotor. The resulting carbonylation mixture comprises the catalyst system, the carboxylic acid carbonylation product and higher boiling (di-)carboxylic acid byproducts. The carboxylic acid is separated from the carbonylation mixture by partial vaporization resulting in that the carboxylic acid is obtained as a gaseous product and the catalyst and the higher boiling (di-)carboxylic acid byproducts remains in the liquid residue. These higher boiling (di-)carboxylic acids tend to form tars in the stream of the recirculating catalyst system.

A disadvantage of this process is that, due to the possible accumulation of tars in the recirculating catalyst system, it is necessary to separate the tar compounds by means of a purge. The presence of a high boiler purge has been found to be a problem because such purge is a potential source of rhodium loss. Rhodium metal is extremely expensive and significant losses can easily make the operation of commercial plants uneconomic. Recovery of the rhodium from such a purge stream is cumbersome and usually involves a number of process steps.

The object of the invention is a process for separating rhodium from mono-carboxylic acids and di-carboxylic acids in a high yield.

This object is achieved in that the following steps are performed
(1) contacting the organic mixture with an amount of water resulting in two separate phases, a first phase being an aqueous phase comprising rhodium and the dicarboxylic acids and a second phase being an organic phase comprising the mono-carboxylic acids, wherein during contacting the molar ratio of halide and rhodium is higher than 1:1,
(2) separating the aqueous phase from the organic phase,
(3) contacting the aqueous phase comprising the rhodium and the dicarboxylic acids with a sufficient amount of an apolar organic extraction solvent resulting in two separate liquid phases, being an aqueous phase comprising the dicarboxylic acids and an apolar organic phase comprising rhodium.

It has been found that with the process of the invention rhodium can be succesfully removed from the organic mixture by extraction. The extraction can be simply operated in a continuous mode on a large scale. Another advantage of the process is that rhodium is separated in a high yield from all the (by-)products formed in the carbonylation process. Another advantage is that, when the separation of the carbonylation product and the catalyst system is performed with the process according to the invention, it is not necessary anymore to perform a high boiler purge.

Another advantage of the process according to the invention is that no distillation of the organic mixture is required in order to separate rhodium from the organic mixture. This is advantageous because the catalyst system comprising rhodium and a halide compound can degradate due to the temperature applied in the distillation.

US-A-4388217 describes a process for recovering rhodium from a high boiler purge formed during the preparation of acetic anhydride (by the rhodium catalyzed carbonylation of a mixture of methyl iodide and methyl acetate and/or dimethyl ether) by means of extracting the rhodium using aqueous hydrogen iodide and methyl iodide, whereby the rhodium is for the greater part extracted into the aqueous phase. This publication does not describe the presence of dicarboxylic acids in the high boiler purge. This process would however not be capable of separating rhodium from the dicarboxylic acids because the latter compounds would also be extracted to the aqueous phase.

The organic mixture comprising rhodium, halide compound, higher boiling mono-carboxylic acids and dicarboxylic acids may be obtained by a carbonylation process in which an alcohol or an olefinic compound is carbonylated to a mono-carboxylic acid in the presence of a rhodium/halogen promotor catalyst system. Exemplary carbonylation processes are described in US-A-5145995 and US-A-3769329.

The invention is therefore directed to a process to separate rhodium from a carbonylation reaction mixture.

The mono-carboxylic acid carbonylation product is preferably a C₄-C₂₅ carboxylic acid and more preferably a C₅-C₁₇ carboxylic acid. The carboxylic acid may be saturated or unsaturated. Examples of possible mono-carboxylic acids are butanoic acid, stearic acid, pentanoic acid, pentenoic acid. A commercially important carboxylic acid is pentenoic acid, which is an intermediate compound to adipic acid (Nylon-6,6-precursor) or ε-caprolactam (Nylon-6-precursor).

The organic mixture to be treated in the process according to the invention may be any mixture comprising rhodium, halide compound, mono-carboxylic acids and dicarboxylic acids. The organic mixture may for example also be, next to the above mentioned carbonylation reaction mixture, a high boiler carbonylation purge stream or a crude product stream of the mono-carboxylic acid carbonylation product. An example of such crude product stream is obtained by the distillation described in WO-A-9614287.

The invention is therefore also directed to a process to separate rhodium from a high boiler carbonylation purge comprising rhodium, halide compound, mono-carboxylic acids, dicarboxylic acids and tar compounds. The high boiler purge is generally removed continuously or intermittently from the carbonylation system. The high boiler purge may be removed either directly from the carbonylation reactor or from some point in the catalyst recycle stream.

It has been found that with the process according to the invention the rhodium can be separated from a high boiler carbonylation purge comprising mono-carboxylic acids, dicarboxylic acids and other higher boiling compounds like tar compounds, in a high yield.

The process according to the invention will be described in more detail for the following preferred embodiment. It shall be evident that the below stated conditions will also be applicable for the above described starting mixtures in a manner clear to one skilled in the art.

The invention is especially directed to a process to separate rhodium from C6-dicarboxylic acids and C9-carboxylic acids. Such mixtures are present in a process to prepare pentenoic acid, in particular 3-pentenoic acid, or adipic acid by carbonylation of butadiene or a butadiene derivative with water in the presence of carbon monoxide and a catalyst system comprising rhodium and a halide promoter compound as for example described in EP-A-405433 or EP-A-428979. These mixtures are for example the reactor effluent, a high boiler purge or a crude pentenoic acid product mixture. Examples of butadiene derivatives are crotyl alcohol, crotyl iodide, crotyl acetate or crotyl pentenoate. The halide compound in the organic starting mixture may be any of the promotors cited in these references. The halide promotor compound is preferably an iodide compound. The iodide promoter is preferably HI or butyl iodide. Most preferably HI is used. When HI is used the mixture will also contain iodide compounds formed by reaction of HI and the compounds present during the carbonylation reaction, for example crotyl iodide and butyl iodide.

The organic starting mixture will generally comprise an organic solvent. The choice of the solvent depends on the specific starting compound to be carbonylated. The organic solvent may be any organic acid, diacid or polyacid of 2 to 20 carbon atoms or may be a mixture of at least two of these acids. The organic solvent should be inert during the carbonylation reaction. A preferred organic solvent is the mixture of acid compounds which are by-products of the carbonylation reaction and which are less volatile than the carboxylic acid carbonylation product. Six carbon dicarboxylic acids and saturated and unsaturated nine carbon mono-carboxylic acids are especially advantageous when the organic starting mixture is derived from a carbonylation process to prepare pentenoic acid because these C6-dicarboxylic acids and C9-carboxylic acids are by-products of this reaction.

The C9-carboxylic acids are for example nonanoic acid, nonenoic acid, branched or cyclic C9-carboxylic acids, for example cyclohexylpropionic acid, or mixtures thereof.

The C6-dicarboxylic acid byproducts are for example adipic acid, 2-methylglutaric acid, ethyl succinic acid, dimethyl succinic acid or mixtures thereof.

The organic starting mixture preferably contains at least rhodium, iodide promotor, C9-monocarboxylic acids and C6-dicarboxylic acids. In case the organic mixture is directly derived form the carbonylation reactor, it contains also a substantial amount of 3-pentenoic acid. In the case the organic mixture is a high boiler carbonylation purge, the organic mixture contains substantial amounts of other higher boiling compounds, for example tar compounds.

In the first step (step (1)) of the process according to the invention, the organic starting mixture is contacted with an amount of water in order to extract rhodium and C6-dicarboxylic acids into the aqueous phase. The C9-mono-carboxylic acids and the optionally present pentenoic acids and tar compounds substantially remain in the organic phase. The amount of water should be sufficiently high in order that two separate liquid phases exist during extraction. However too much water will result in that less C9-mono-carboxylic acids and optionally present pentenoic acids and tar compounds will remain in the organic phase. The volumetric ratio water and organic phase is preferably between 0.1 and 10. The water may contain salts or other compounds as long as these do not substantially interfere with the first step of the process of the invention.

The amount of organic compounds in the organic starting mixture should be sufficiently high in order to get a sufficient extraction efficiency in the first step of the process according to the invention. If the concentration of organic compounds is too low, the organic starting mixture has to be concentrated by for example evaporating the volatile compounds. If rhodium is for example to be removed from a carbonylation mixture, it can be necessary to adjust the composition of the organic starting mixture before performing the extraction step (1) according to the invention. The adjustment of the composition is necessary in order to achieve the preferred range of concentration of organic compounds as described above

The halide (as inorganic halide) compound to rhodium molar ratio in the organic starting mixture is generally between 0.5:1 and 20:1 and more in particular between 1:1 and 5:1. To achieve the desired rhodium/halide compound ratio's in the extraction step (1) additional halide compound will generally have to be added. Preferably the additional halide compound and the water are added as a mixture. Preferably the halide compound added is the same as the halide compound present in the organic mixture. It has been found to be critical to add sufficient halide compound in order to transfer rhodium to the aqueous phase and at the same time not to add too much water. Because of the high affinity of inorganic halide compounds, for example HI, for water most of these halide compounds will be present in the resulting aqueous extract. The molar ratio halide compound and rhodium is preferably higher than 5:1 and more preferably higher than 10:1. The upper limit is not critical and will practically be limited by the solubility of the halide compound in water.

In case the organic starting mixture is derived from a carbonylation reactor, the organic raffinate of step (1) contains next to the C9-mono-carboxylic acids a substantial amount of pentenoic acid(s). The pentenoic acid can be simply isolated from the organic raffinate obtained in step (1) by means of distillation. The distillation residue will consist mainly of C9-mono-carboxylic acids which can be reused as organic solvent in the carbonylation reaction.

In case the organic starting mixture is a high boiler carbonylation purge, the organic raffinate of step (1) contains next to the C9-mono-carboxylic acids an amount of tar compounds. The C9-mono-carboxylic acids are preferably removed from the organic raffinate before it is deposed of. This separation is preferably performed by distillation. The C9-mono-carboxylic acids can be reused as organic solvent in the carbonylation reaction. The distillation residue will consist mainly of tar compounds which can be disposed of.

In step (2) of the process according to the invention, the aqueous extract is separated from the organic raffinate.

The aqueous extract obtained in step (1) is subsequently contacted with a sufficient amount of an apolar organic solvent in step (3) of the process according to the invention. In order to get a high extraction efficiency of rhodium from the aqueous phase to the organic phase in step (3) of the invention, the molar ratio of rhodium to halide (as inorganic halide) compound in step (3) of the process is preferably between 1:1 and 1:5. To achieve the desired rhodium/halide compound ratio's in the extraction step (3), it can be necessary to remove some amount of the present halide compound from the aqueous extract obtained in step (1). The mode of separation of halide compound from the aqueous extract is not critical and can be performed using for example distillation, absorbing or ion exchanging. This separation of halide compound from the aqueous extract is preferably performed by distillation in the presence of carbon monoxide.

The extraction solvent of step (3) is an apolar, organic solvent which is substantially immiscible with water in order that two phases will exist during and after the extraction. Some mutual solubility of water and the extraction solvent is allowed and does not necessarily have a negative influence on the practical application of this invention. The organic extraction solvent should have substantially apolar characteristics (relative to water) in which the (polar) dicarboxylic acid compounds are substantially insoluble and is preferably inert under carbonylation conditions. Examples of possible extraction solvents are aromatic solvents; substituted hydrocarbons; unsubstituted hydrocarbons, preferably containing more than 4 carbon atoms. Examples of suitable aromatic solvents are benzene and toluene. Examples of suitable hydrocarbons are methane, pentane, hexane or carbontetrachloride.

Preferably compounds which can form a complex with rhodium are used as apolar organic extraction solvent of step (3). Suitable complex forming agents are hydrocarbons containing at least one olefinic double bond and organic compounds containing phosphorous and/or nitrogen. Examples of hydrocarbons containing at least one olefinic double bond are alkenes, preferably alkenes with 2 to 15 carbon atoms; linear or cyclic alkadienes, preferably alkadienes with 2 to 15 carbon atoms. Examples of preferred alkenes are ethylene, propylene, isobutylene, 1-butene, 2-butene, 1-pentene, 2-pentene, hexene, heptene, octene, nonene, decene, 3-methyl-1-butene, 2-methyl-2-butene or 2,3-dimethyl-2-butene. Examples of preferred alkadienes are 1,2-butadiene, 1,3-butadiene, 1,3-pentadiene, 1,4-pentadiene, hexadiene, isoprene, 2,3-dimethyl-1,3-butadiene, 1,2-propadiene, vinyl acetylene, cyclopentadiene, cyclohexadiene, cyclooctadiene or cyclodecadiene. Suitable complex forming compounds containing phosphorous are organic phosphines or diphosphines which are preferably solubilized in a suitable apolar, organic solvent. Examples of organic phosphines and diphosphines are arylphosphines like triphenylphosphine and aryldiphosphines. Suitable complex forming compounds containing nitrogen are primary, secundary or tertiary amines which are substantially not soluble in water. Examples of suitable complex forming agents are pyridine and alkylamines containing at least two carbon atoms, for example butylamine, pentylamine and octylamine.

The amount of apolar organic extraction solvent will depend on the partition coëfficient which is dependent on the organic extraction solvent.

The rhodium and at least a part of the halide promotor compound can be simply isolated from the apolar organic extract by for example distillation. The rhodium and the halide promotor will be present in the distillation residue. The distillate will consist mainly of apolar organic extraction solvent which can be reused in the extraction of step (3) according the invention. The residue containing rhodium and the halide promotor compound can be reused in the carbonylation reaction. The distillation is preferably performed in the presence of carbon monoxide in order to avoid any rhodium precipitation.

A preferred embodiment of the invention is a process in which the olefinic starting compound of the carbonylation is used as extraction solvent in step (3). For example in a process to prepare pentenoic acid butadiene is advantageously used. This is advantageous because the apolar organic extract obtained in step (3) of the process according to the invention can be directly used in the carbonylation reaction without having to first isolate rhodium and optionally the halogen compound.

The C6-dicarboxylic acids in the aqueous raffinate obtained in step (3) are preferably separated.

During the extractions (step (1) and (3)) the liquid phases are preferably saturated with carbon monoxide in order to avoid any rhodium precipitation. The pressure is not critical but should be sufficient to maintain liquid phases. Preferably atmospheric pressures are applied for practical reasons. The temperature is equal or preferably lower than the carbonylation reaction temperature. The temperature of a reaction mixture leaving the carbonylation reactor is generally between 100 and 150°C. More preferably the temperature during the extraction is between about room temperature and about 100°C. Most preferably the temperature is between 30 and 60 °C. The lower temperature limit is not critical, provided that the temperature is sufficiently high in order to keep the compounds in the fluid state.

The process according to the invention is preferably performed continuously. The contacting can be performed with the usual liquid-liquid contactors, for example a series of mixer-settlers or pulsed beds. The process is preferably performed in at least two counter currently operated, vertically placed vessels, operated in series. To the top of the first vessel the water is fed and to the bottom the organic starting mixture is fed. The aqueous extract and the organic raffinate are obtained at the bottom and top of the column respectively. To the top of the second vessel the aqueous extract of the first vessel is fed and to the bottom the apolar extraction solvent. The aqueous phase and the organic phase are obtained at the bottom and top of the column respectively. The column has preferably more than three theoretically exchange stages.

The invention is especially directed to a process to continuously prepare pentenoic acid by carbonylation of butadiene or a butadiene derivative and water in the presence of a catalyst system comprising rhodium and a HI promotor compound and an organic solvent comprising C6-dicarboxylic acids and C9-mono-carboxylic acids in which the following steps are performed,
(a) separating rhodium from the carbonylation reactor effluent with the process according to the invention resulting in an organic raffinate comprising pentenoic acid and C9-monocarboxylic acids, an organic extract comprising rhodium and iodide compound and an aqueous raffinate comprising C6-dicarboxylic acids,
(b) isolating the pentenoic acid from the organic raffinate by distillation and recycling part of the C9-mono-carboxylic acids to the carbonylation reactor,
(c) isolating the rhodium an at least a part of the iodide compound from the organic extract by distillation and
(d) recycling the rhodium and iodide compound to the carbonylation reactor.

### Example

To a reaction mixture containing 1070 ppm rhodium and hydrogen iodide (molar ratio I/Rh=2) in 55 grams nonanoic acid, 27 grams of a mixture of dicarboxylic acids (ethylsuccinic acid, methylglutaric acid, and adipic acid) an amount of 8 grams of water was added at room temperature under an atmosphere of CO.

The obtained mixture was extracted with 20 ml of a mixture of 15% nonanoic acid, 55% dicarboxylic acids, 30% water and 0.2 % HI. After phase separation the water phase was isolated. This procedure was repeated five more times. All aqueous phases were combined resulting in a phase containing 820 ppm rhodium.
The combined aqueous phase was split in two of which one phase was reextracted with cyclooctadiene and the other with butadiene. The results are listed in the Table below:

| Rhodium contents in waterphase (ppm) | | |
|---|---|---|
| extraction no. | 4 extractions with 27 g cyclooctadiene each | 3 extraction's with 15 g butadiene each |
| 0 | 820 | 820 |
| 1 | 200 | 384 |
| 2 | 87 | 118 |
| 3 | 39 | 33 |
| 4 | 11 | |

## Claims

1. Process to separate rhodium from an organic mixture comprising mono-carboxylic acids, dicarboxylic acids, rhodium and a halide compound, characterized in that the following steps are performed
(1) contacting the organic mixture with an amount of water resulting in two separate phases, a first phase being an aqueous phase comprising rhodium and the dicarboxylic acids and a second phase being an organic phase comprising the mono-carboxylic acids, wherein during contacting the molar ratio of halide and rhodium is higher than 1:1,
(2) separating the aqueous phase from the organic phase,
(3) contacting the aqueous phase comprising the rhodium and the dicarboxylic acids with a sufficient amount of an apolar organic extraction solvent resulting in two separate liquid phases, being an aqueous phase comprising the dicarboxylic acids and an apolar organic phase comprising rhodium.

2. Process according to claim 1, characterized in that the molar ratio of halide and rhodium in step (1) is higher than 5:1.

3. Process according to claim 2, characterized in that the molar ratio of halide and rhodium in step (1) is higher than 10:1.

4. Process according to any one of claims 1-3, characterized in that the molar ratio of rhodium to halide in step (3) is between 1:1 and 1:5.

5. Process according to any one of claims 1-4, characterized in that the organic mixture is the reaction mixture of the carbonylation of an olefinic starting compound and water catalyzed by a rhodium/halide catalyst system or is a high boiler carbonylation purge.

6. Process according to any one of claims 1-5, characterized in that the extraction solvent in step (3) is a complex forming agent for rhodium.

7. Process according to claim 6, characterized in that the complex forming agent is a hydrocarbon containing at least one olefinic double bond.

8. Process according to claim 7, characterized in that the extraction solvent in step (3) is the olefinic starting compound of the carbonylation.

9. Process according to any one of claims 1-8, characterized in that the halide promotor is an iodide compound.

10. Process according to claim 9, characterized in that the iodide compound is HI.

11. Process to continuously prepare pentenoic acid by carbonylation of butadiene or a butadiene derivative and water in the presence of a catalyst system comprising rhodium and a HI promotor and an organic solvent comprising C6-dicarboxylic acids and C9-mono-carboxylic acids, characterized in that the following steps are performed,
(a) separating rhodium from the carbonylation reactor effluent with the process according to claims 1-10 resulting in an organic raffinate comprising pentenoic acid and C9-monocarboxylic acids, an organic extract comprising rhodium and iodide compound and an aqueous raffinate comprising C6-dicarboxylic acids,
(b) isolating the pentenoic acid from the organic raffinate by distillation and recycling part of the C9-mono-carboxylic acids to the carbonylation reactor,
(c) isolating the rhodium an at least a part of the iodide compound from the organic extract by distillation and
(d) recycling the rhodium and iodide compound to the carbonylation reactor.

12. Process according to claim 11, characterized in that the organic solvent in step (3) of stage (a) is butadiene and stage (c) is omitted.
